# EUROPÄISCHE PATENTSCHRIFT

(11) **EP 1 201 259 B1**
(45) Veröffentlichungstag und Bekanntmachung des Hinweises auf die Patenterteilung: **08.12.2004**
(21) Anmeldenummer: 01117612.0
(22) Anmeldetag: 23.07.2001
(51) Int. Cl.: A61M 15/00, A61M 16/00

(54) **Inhalationsbeutel**
Bag for inhalation
Sac pour inhalation

(30) Priorität: 31.10.2000 DE 20018588 U
(43) Veröffentlichungstag der Anmeldung: 02.05.2002
(73) Patentinhaber: Institut für Aerosol-Medizin InAMed GmbH, 82131 Gauting (DE)
(72) Erfinder: Roeder, Sascha, 85053 Ingolstadt (DE); Müllinger, Bernard, 80796 Gauting (DE)
(74) Vertreter: Reinhard - Skuhra - Weise & Partner

(56) Entgegenhaltungen:
- DE-A- 19 859 177
- GB-A- 2 301 040
- US-A- 3 363 833
- US-A- 5 787 880

## Beschreibung

Die Erfindung betrifft eine Vorrichtung zur Aufnahme und Abgabe eines Aerosols zu Inhalationszwecken gemäß dem Oberbegriff des Patentanspruchs 1.

Die inhalatorische Medikamentenapplikation gewinnt zunehmend an Bedeutung, da damit eine unmittelbare und wirkungsvolle Verabreichung gezielter Medikamentendosen möglich ist. Zu den Faktoren, die eine exakte Deposition von Medikamenten nach Depositionsort und Dosis bestimmen, gehören die Aufbereitung des Aerosols und die Festlegung eines bestimmten Volumens. Für die Aerosolaufbereitung ist es bisher üblich, entleerte Aufnahmeeinrichtungen mit unter Druck befindlicher Luft zu befüllen, welche beim Durchtritt durch eine vorgeschaltete Einrichtung Medikamentenpulver mitreißt.

Eine Vorrichtung der eingangs genannten Gattung ist bereits in der US 3,363,833 offenbart. Diese vorbekannte Vorrichtung besitzt einen elastischen elypsoidförmigen Beutel mit Materialschwächungen aufweisenden Wandabschnitten zum Erleichtern des Zusammenschiebens und ein Einlaßventil und ein Auslaßventil zum Einpumpen von Luft in die Lunge eines Patienten.

Der Erfindung die Aufgabe zugrunde, eine Vorrichtung der eingangs der genannten Gattung verfügbar zu machen, die eine einfache Aufbereitung eines Medikamentenaerosols und eine dosierte inhalatorische Applikation von Medikamenten ermöglicht.

Erfindungsgemäß wird diese Aufgabe durch die im Anspruch 1 angegebenen Merkmale gelöst. Bevorzugte Merkmale, die die Erfindung vorteilhaft weiterbilden, sind den untergeordneten Ansprüchen zu entnehmen.

Gemäß einer bevorzugten Ausgestaltung der Erfindung ist die Auffalteinrichtung mit wenigsten zwei großflächigen formstabilen Abschnitten gebildet, die miteinander über elastische Spreizelemente verbunden sind. In einer vorzugsweisen Ausgestaltung sind dabei zwei großflächige einander gegenüberliegende formstabile Abschnitte vorgesehen, die an seitlichen Rändern mit den Spreizelementen verbunden sind, wobei diese Ränder bevorzugt parallel zu der Längsachse der Aufnahmeeinrichtung angeordnet sind.

Die Spreizelemente bestehen ebenso wie die großflächigen Wandabschnitte bevorzugt aus einem für die Kontaktierung mit Arzneimitteln zugelassenen elastomeren Kunststoff, wie Silikon oder dergleichen. Für ein seitliches Einknicken der beutelförmigen Aufnahmeeinrichtung sind dabei nach einer bevorzugten weiteren Ausgestaltung der Erfindung die Spreizelemente mit Materialschwächungen, insbesondere in Form von seitlichen Ausnehmungen und Löchern, versehen.

Nach einer weiteren bevorzugten Ausgestaltung der Erfindung sind die großflächigen Wandabschnitte rechteckig und flach ausgebildet und im Bereich ihrer Ecken mit blattförmigen Spreizelementen verbunden, wobei die Aufnahmeeinrichtung im Bereich der Zugangsöffnung eine formstabile Frontplatte aufweist.

Alternativ können die großflächigen Wandabschnitte und/oder die Spreizelemente einstückig mit der Aufnahmeeinrichtung gebildet sein.

Nachfolgend wird die Erfindung an Hand eines Ausführungsbeispiels unter Bezugnahme auf die beigefügten Figuren näher erläutert. Es zeigen:
- Figur 1: eine Seitenansicht auf ein Ausführungsbeispiel einer erfindungsgemäßen Vorrichtung; und
- Figur 2: eine Draufsicht auf das in Figur 1 dargestellte Ausführungsbeispiel.

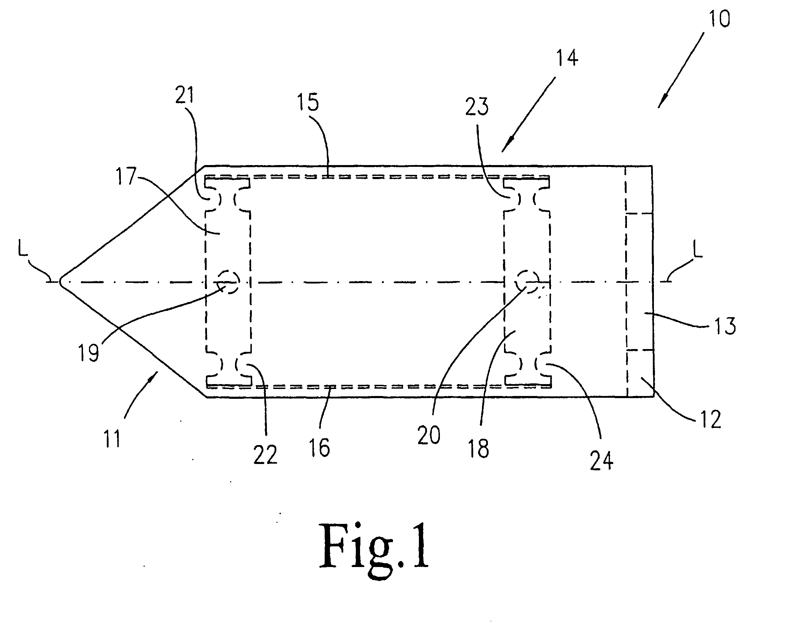

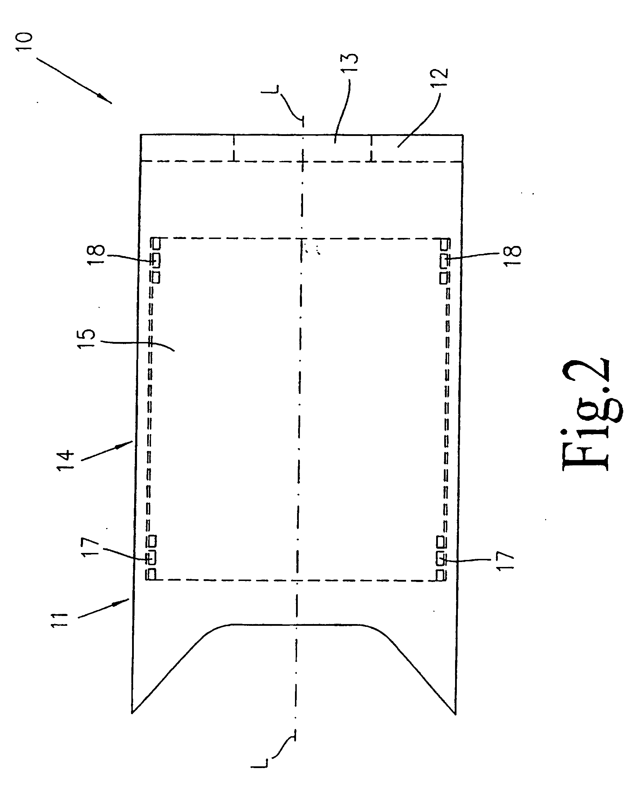

In den Figuren 1 und 2 ist eine Ausgestaltung einer erfindüngsgemäßen Vorrichtung 10 zur Aufnahme und Abgabe eines Aerosols zu Inhalationszwecken dargestellt. Die Vorrichtung 10 besteht aus einer beutelförmigen Aufnahmeeinrichtung 11, die mit einem gasdichten zusammenfaltbaren Material, wie beispielsweise Kunststofffolie, gebildet ist. Die Aufnahmeeinrichtung 11 besitzt eine Längsachse L und ist in den Figuren 1 und 2 linksseitig geschlossen, während sich rechtsseitig eine Frontplatte 12 befindet, mit der die beutelförmige Aufnahmevorrichtung 11 verbunden ist. In der Frontplatte 12 ist im Bereich der Längsachse L eine Zugangsöffnung 13 vorgesehen, durch die die Aufnahmeeinrichtung 11 mit Aerosol befüllbar und die Aerosolfüllung aus der Aufnahmeeinrichtung inhalierbar ist.

Als Besonderheit weist die erfindungsgemäße Vorrichtung eine integrierte Auffalteinrichtung 14 auf, durch die die Aufnahmeeinrichtung aus einem vorbestimmten Einfaltzustand in einen stabilen Auffaltzustand bringbar ist. Die Auffalteinrichtung 14 besteht aus zwei flachen rechteckförmigen. Kunststoffplatten 15 und 16, die als großflächige Wandabschnitte einander gegenüberliegen und an der Innenwandung der Aufnahmeeinrichtung 11 anliegen. Die formstabilen Wandabschnitte 15 und 16 werden durch randseitig befestigte blattförmige Silikonfedern 17 und 18 auseinandergedrückt und beaufschlagen damit großflächig gegenüberliegende Innenflächen der beutelförmigen Aufnahmeeinrichtung 11, wodurch eine selbstaufklappende Wirkung erreicht wird.

Die blattförmigen Silikonfedern 17 und 18 weisen Materialschwächungen 19 - 24 auf, die ein definiertes seitiges Einknicken der beutelförmigen Aufnahmeeinrichtung 11 ermöglichen.

Die Auffalteinrichtung 14 kann alternativ in nicht dargestellter Weise auch einstückig mit der beutelförmigen Aufnahmeeinrichtung 11 gebildet sein.

Für ein Befüllen der beutelförmigen Aufnahmeeinrichtung 11 wird diese zunächst in ihren definierten zusammengefalteten Zustand gebracht, wobei sie auf der Ober- und Unterseite durch die eingesetzte Auffalteinrichtung 14 stabilisiert wird und nur seitlich einknicken kann. Für das vorgesehene Selbstaufklappen sind die blattförmigen Silikonfedern 17 und 18 verantwortlich, wobei durch die einströmende Luft beim Aufklappen der beutelförmigen Aufnahmeeinrichtung 11 Medikamentenpulver zur Herstellung des gewünschten Aerosols in das Innere zerstäubt werden können.

Nach einer weiteren nicht dargestellten Ausgestaltung der Erfindung können für die stabilisierenden formstabilen Wandabschnitte 15 und 16 noch zusätzliche Lagesicherungen vorgesehen oder einstückig mit der Aufnahmeeinrichtung gebildet sein, um das Ein- und Ausfaltverhalten in den jeweils vorgesehenen Bereichen exakt zu steuern.

## Patentansprüche

1. Vorrichtung zur Aufnahme und Abgabe eines Aerosols zu Inhalationszwecken, mit einer beutelförmigen Aufnahmeeinrichtung (11) aus einem gasdichten zusammenfaltbaren Material, und mit einer Ein- und Austrittsöffnung (13) für das Aerosol, die an der Aufnahmeeinrichtung (11) vorzugsweise in deren Längsachse (L) gebildet ist und durch die die Aufnahmeeinrichtung (11) mit Aerosol befüllbar und die Aerosolfüllung aus der Aufnahmeeinrichtung (11) inhalierbar ist; **gekennzeichnet durch** eine Auffalteinrichtung (14), die mit Wandabschnitten (15, 16) zum federnd vorgespannten Einnehmen und Stabilisieren des Auffaltzustandes der Aufnahmeeinrichtung (11) gebildet ist und **durch** die die Aufnahmeeinrichtung (11) zum druckluftfreien Aerosolaufbereiten und Befüllen mit Aerosol **durch** die Ein- und Auslaßöffnung (13) aus einem vorbestimmten Einfaltzustand in einen stabilen Auffaltzustand selbständig bringbar ist.

2. Vorrichtung nach Anspruch 1, **dadurch gekennzeichnet, daß** die Wandabschnitte (15, 16) und die Spreizelemente (17, 18) aus Kunststoff bestehen.

3. Vorrichtung nach Anspruch 2, **dadurch gekennzeichnet, daß** die Spreizelemente (17, 18) für ein seitliches Einknicken der beutelförmigen Aufnahmeeinrichtung (11) ausgebildet sind.

4. Vorrichtung nach einem der Ansprüche 1 bis 3, **dadurch gekennzeichnet, daß** die Wandabschnitte (15, 16) und/oder die Spreizelemente (17, 18) einstückig mit der Aufnahmeeinrichtung (11) gebildet sind.

5. Vorrichtung nach einem der vorangehenden Ansprüche, **dadurch gekennzeichnet, daß** die Auffalteinrichtung (14) mit wenigstens zwei großflächigen formstabilen Wandabschnitten (15, 16) gebildet ist, die miteinander über elastische Spreizelemente (17, 18) verbunden sind.

6. Vorrichtung nach Anspruch 5, **dadurch gekennzeichnet, daß** die großflächigen formstabilen Wandabschnitte an gegenüberliegenden seitlichen Rändern mit den Spreizelementen (17, 18) verbunden sind.

7. Vorrichtung nach Anspruch 6, **dadurch gekennzeichnet, daß** die seitlichen Ränder der großflächigen Wandabschnitte parallel zu der Längsachse (L) der Aufnahmeeinrichtung (11) angeordnet sind.

8. Vorrichtung nach einem der Ansprüche 5 bis 7, **dadurch gekennzeichnet, daß** die großflächigen Wandabschnitte (15, 16) rechteckig und flach ausgebildet und im Bereich ihrer Ecken mit blattförmigen Spreizelementen (17, 18) verbunden sind, wobei die Aufnahmeeinrichtung (11) im Bereich der Zugangsöffnung (13) eine formstabile Frontplatte (12) aufweist.

9. Vorrichtung nach einem der vorangehenden Ansprüche, **dadurch gekennzeichnet, daß** die Auffalteinrichtung (14) in der Aufnahmeeinrichtung (11) vorgesehen ist.

10. Vorrichtung nach einem der vorangehenden Ansprüche, **dadurch gekennzeichnet, daß** die beutelförmige Aufnahmeeinrichtung (11) einstückig ausgebildet ist.

## Claims

1. Device for absorbing and dispensing an aerosol for purposes of inhalation, which comprises a bag-shaped absorbing device (11) of a gastight foldable material and an inlet and outlet hole (13) for aerosol being formed at the absorbing device (11) preferably in the longitudinal direction (L) thereof and through which the absorbing device (11) can be filled with aerosol and the aerosol filling can be inhaled from the absorbing device (11); **characterized by** an unfolding device (14), which is formed with wall portions (15, 16) for elastically biased adopting and stabilizing the unfolded state of the absorbing device (11), and by means of which the absorbing device (11) for the preparation of aerosol without compressed air and filling with aerosol through the inlet and outlet hole (13) independently can be brought from a predetermined folded-up state to an unfolded state.

2. Device according to claim 1, **characterized in that** the wall portions (15, 16) and the spreaders (17, 18) are of plastics.

3. Device according to claim 2, **characterized in that** the spreaders (17, 18) are developed for lateral buckling of the bag-shaped absorbing device (11).

4. Device according to claim 1 to 3, **characterized in that** the wall portions (15, 16) and/or the spreaders (17, 18) are integrally formed with the absorbing device (11).

5. Device according to one of the preceding claims, **characterized in that** the unfolding device (14) is formed with at least two large surface dimensionally stable wall portions (15, 16), which are connected to each other via elastic spreaders (17, 18).

6. Device according to claim 5, **characterized in that** the large surface dimensionally stable wall portions are connected to said spreaders (17, 18) at the opposite lateral edges.

7. Device according to claim 6, **characterized in that** the lateral edges of the large surface wall portions are arranged parallel to the longitudinal axis (L) of the absorbing device (11).

8. Device according to one of the claims 5 to 7, **characterized in that** the large surface wall portions (15, 16) are developed in a rectangular and flat manner and connected to laminated spreaders (17, 18) in the region of the corners thereof, wherein said absorbing device (11) has a dimensionally stable front plate (12) in the region of the access hole (13).

9. Device according to one of the preceding claims, **characterized in that** the unfolding device (14) is provided in the absorbing device (11).

10. Device according to one of the preceding claims, **characterized in that** the bag-shaped absorbing device (11) is formed integrally developed.

## Revendications

1. Dispositif de réception et de dégagement d'un aérosol à des fins d'inhalation, comprenant un sac (11) de réception en une matière pliable et étanche au gaz et comprenant une ouverture (13) d'entrée et de sortie de l'aérosol, qui est formée sur le sac (11) de réception, de préférence dans son axe (L) longitudinal, et par laquelle le sac (11) de réception peut être empli d'aérosol et la charge d'aérosol peut être inhalée en sortant du sac (11) de réception, **caractérisé par** un dispositif (14) de dépliage, qui est formé en ayant des parties (15, 16) de paroi pour faire prendre, avec mise sous précontrainte élastique, au sac (11 ) de réception l'état déplié et l'y stabiliser et par lequel le sac (11) de réception peut, pour préparer de l'aérosol sans air comprimé et emplir d'aérosol par l'ouverture (13) d'entrée et de sortie, passer automatiquement d'un état replié déterminé à l'avance à un état déplié stable.

2. Dispositif suivant la revendication 1, **caractérisé en ce que** les parties (15, 16) de paroi et les éléments (17, 18) d'écartement sont en matière plastique.

3. Dispositif suivant la revendication 1, **caractérisé en ce que** les éléments (17, 18) d'écartement sont constitués pour permettre de comprimer latéralement le sac (11) de réception.

4. Dispositif suivant l'une des revendications 1 à 3, **caractérisé en ce que** les parties (15, 16) de paroi et/ou les éléments (17, 18) d'écartement sont d'un seul tenant avec le sac (11) de réception.

5. Dispositif suivant l'une des revendications précédentes, **caractérisé en ce que** le dispositif (14) de dépliage est formé en ayant au moins deux grandes parties (15, 16) de paroi de grande surface et de forme stable, qui sont reliées entre elles par des éléments (17, 18) élastiques d'écartement.

6. Dispositif suivant la revendication 5, **caractérisé en ce que** les parties de paroi de grande surface et de forme stable sont reliées par des bords latéraux opposés aux éléments (17, 18) d'écartement.

7. Dispositif suivant la revendication 6, **caractérisé en ce que** les bords latéraux des parties de paroi de grande surface sont parallèles à l'axe (L) longitudinal du sac (11) de réception.

8. Dispositif suivant l'une des revendications 5 à 7, **caractérisé en ce que** les parties (15, 16) de paroi de grande surface sont rectangulaires et plates et sont reliées dans la zone de leurs sommets à des éléments (17, 18) d'écartement en forme de lame, le sac (11) de réception ayant une plaque (12) avant de forme stable dans la zone de l'ouverture (13) d'accès.

9. Dispositif suivant l'une des revendications précédentes, **caractérisé en ce que** le dispositif (14) de dépliage est prévu dans le sac (11) de réception.

10. Dispositif suivant l'une des revendications précédentes, **caractérisé en ce que** le sac (11) de réception est d'un seul tenant.
